# EUROPÄISCHE PATENTANMELDUNG

(11) **EP 2 887 065 A1**
(43) Veröffentlichungstag der Anmeldung: **24.06.2015**
(21) Anmeldenummer: 13197895.9
(22) Anmeldetag: 17.12.2013
(51) Int. Cl.: G01N 33/497, G01N 1/40

(54) **Verfahren zur Atemgasanalyse bei infektiösen Erkrankungen**

(71) Anmelder: Forest Laboratories Deutschland GmbH, 10117 Berlin (DE)
(72) Erfinder: Die Erfindernennung liegt noch nicht vor
(74) Vertreter: Meinken, Claudia

(57) **Zusammenfassung**

Die vorliegende Erfindung betrifft ein Verfahren zur Atemgasanalyse sowie die Verwendung dieses Verfahrens und ein entsprechendes Diagnostikum, das Mittel zur Durchführung des erfindungsgemäßen Verfahrens umfasst. Dabei werden die in der ausgeatmeten Luft eines Individuums enthaltenen flüchtigen organischen Substanzen (VOCs) in einer Needle Trap Device (NTD) angereichert und einzelne VOCs, die für eine Erkrankung, beispielsweise eine infektiöse Erkrankung oder eine Tumorerkrankung, vorzugsweise eine infektiöse Lungenerkrankungen charakteristisch sind, in der angereicherten Luft nachgewiesen.

## Beschreibung

Die vorliegende Erfindung betrifft ein Verfahren zur Atemgasanalyse sowie die Verwendung dieses Verfahrens und ein entsprechendes Diagnostikum, das Mittel zur Durchführung des erfindungsgemäßen Verfahrens umfasst. Dabei werden die in der ausgeatmeten Luft eines Individuums enthaltenen flüchtigen organischen Substanzen (VOCs) in einer Needle Trap Device (NTD) angereichert und einzelne VOCs, die für eine Erkrankung, beispielsweise eine infektiöse Erkrankung oder einer Tumorerkrankung, vorzugsweise eine infektiöse Lungenerkrankungen charakteristisch sind, in der angereicherten Luft mittels Gaschromatographie-Massenspektrometrie (GC-MS) nachgewiesen.

Bei der Atemgasanalyse wird die Ausatemluft eines Probanden untersucht. Es ist bekannt, dass in der ausgeatmeten Luft spezifische Krankheitsmarker und Metabolite von Medikamenten und Stoffwechselprozessen gefunden werden können. Dabei handelt es sich um kurz- und mittelkettige Kohlenwasserstoffe bis hin zu Hormonen und körpereigenen Mediatorstoffen. Diese Stoffe werden unter dem Begriff "flüchtige organische Substanzen" (engl. volatile organic compounds (VOCs)) subsumiert.

VOCs werden von der World Health Organization (WHO) als organische Verbindungen mit einem Siedepunkt von 50-100 °C bis 240-260 °C definiert. VOCs sind typische Produkte organischer Prozesse wie z.B. von Verbrennungsvorgängen und können in sehr geringen Konzentrationen in der Umgebungsluft nachgewiesen werden. Die Konzentrationen vieler Substanzen im Atemgas liegen in Bereichen zwischen nmol/l bis pmol/l oder ppb bis ppt im Bezug auf das Volumen (pptV beziehungsweise ppbV). In diesem Zusammenhang konnten bisher mehr als 500 flüchtige organische Substanzen identifiziert werden, die sowohl aus der Umgebung (exogene Substanzen) als auch im Körper gebildet werden (endogene Substanzen). Um metabolische oder pathologische Prozesse zu verfolgen, müssen endogene Substanzen beobachtet werden. Zu den wichtigsten endogenen VOCs im Atemgas zählen C2-C6 Alkane, Alkene, Aldehyde, Ketone und Alkohole.

Bisherige Untersuchungen erfolgten zumeist am Atemkondensat (EBC). Das Atemkondensat wird durch Kondensation infolge Kühlung der Ausatemluft auf - 20°C gewonnen. Dadurch gelingt es, den Wasserdampf in Wasser zu überführen und die aus der Lungentiefe beförderten Aerosoltröpfchen durch Adhäsion an der Wandung der Kühlfalle zu binden. Für die Gewinnung der Atemkondensat-Probe atmet der Proband in Ruhe für eine Dauer von 20 Minuten über ein Mundstück durch ein Nichtrückstromventil, in dem die Ein- und Ausatemluft separiert wird. Methodische Probleme standen einer routinemäßigen Anwendung dieses diagnostischen Verfahrens in der klinischen Praxis bisher entgegen. So ist die Konzentration der VOCs im Atemkondensat im Wesentlichen abhängig von der Feuchtigkeit der Ausatemluft.

Die Ausatemluft kann beispielsweise auch in Beuteln wie Tedlar® Bags gesammelt und aufbewahrt werden. Der konventionelle Transport der Proben mittels Tedlar® Bags und deren Analyse sind aber fehleranfällig, sowie zeit- und kostenintensiv.

Die gewonnene Ausatemluft wird anschließend analysiert, um einzelne VOCs in der ausgeatmeten Luft zu detektieren. Hierbei wurden zumeist die Verfahren der Gaschromatographie (GC) und der Massenspektrometrie (MS) eingesetzt, um einzelne exhalierte Substanzen zu typisieren. Dabei wird die Gaschromatographie vorgeschaltet, um die Substanzen aufzutrennen, bevor sie im Massenspektrometer identifiziert werden. Auf Grund der Größe des Gerätes und des technischen Aufwandes sind massenspektrometrische Untersuchungen nur in einem Labor und die Bedienung nur durch Fachpersonal möglich.

Die Ionenmobilitätsspektrometrie (IMS) gestattet die Untersuchung exhalierter VOCs, ohne diese zunächst weiter zu spezifizieren. Hierbei werden die Moleküle im Exhalat ionisiert und diese Ionen bewegen sich gegen die Strömungsrichtung eines Gases auf eine Elektrode hin und werden hierbei in Abhängigkeit von der Strömungsgeschwindigkeit aufgetrennt. Bei infektiösen Lungenerkrankungen konnten mit der IMS unterschiedliche Bakterien differenziert werden, weil sie ein unterschiedliches Muster von Peaks in den IMS Spektren erzeugen. Die jeweiligen Peaks, die für eine infektiöse Lungenerkrankungen charakteristisch sind, sind jedoch nur indirekt mittels einer Referenzbibliothek zu identifizieren. Diese Probleme stehen einer breiten diagnostischen Anwendung der IMS zum Nachweis und zur Verlaufskontrolle bei infektiösen Lungenkrankheiten entgegen.

Die Tatsache, das in der Ausatemluft viele verschiedene VOCs vorkommen, wobei nur wenige davon für eine bestimmte Krankheit charakteristisch sind, sowie die sehr geringen Konzentrationen einzelner VOCs (im Bereich von ppt-ppb bzw. pptV-ppbV) stellen ein besonderes technisches Problem dar. Erschwerend kommt noch die leichte Flüchtigkeit, d.h. der niedrige Siedepunkt, der VOCs hinzu. Durch die leichte Flüchtigkeit kann die Konzentration der VOCs in der Ausatemluft noch einmal sinken oder es kann sehr leicht zu Konzentrationsverschiebungen einzelner VOCs in der ausgeatmeten Luft kommen.

Nur mittels Präkonzentration der VOCs können überhaupt die nötigen Nachweisgrenzen von Analyseverfahren, wie zum Beispiel der Gaschromatographie, erreicht werden. Seit 1990 wurden daher Methoden zur Probennahme und Analyse von VOCs entwickelt - von Solvent-Extraktionen hin zu lösungsmittelfreien Extraktionen und auch die Präkonzentration mittels Needle Trap Device (NTD).

Ein Needle Trap Device (NTD), auch einfach Needle Trap genannt, ist ein Extraktionsgerät, dass aus einer kleinen Nadel besteht, die ein bis drei Adsorbentien enthält. In die NTD können mittels einer Spritze Flüssigkeiten oder Gase eingezogen und ausgestoßen werden. Analyten können auch passiv durch Diffusion in die Nadel gelangen. Die NTD ist ein Gerät, dass normalerweise zur Probenvorbereitung oder als Hilfsmittel zur Einführung der Probe in ein anderes Gerät, verwendet wird. Sowohl Analyten, die in Flüssigkeiten gelöst sind, als auch Partikel aus Gasen können in der Nadel eingefangen und dort an den Adsorbentien angereichert werden. Dabei werden geringe Volumina (5-100 ml) auf die mit Adsorbentien gefüllte Nadel aufgezogen und anschließend im Analysesystem thermisch desorbiert.

NTDs wurden im Bereich der Umweltanalytik von Eom et al. (Chromatogr. A 2008, 1196-1197, 3-9) zur Anreicherung von BTEX und höheren Alkanen (C₆ - C₁₅) verwendet.

Das Adsorptionsverhalten von Needle Traps wurde mit Hilfe von Gasstandards aus Kohlenwasserstoffen, Aldehyden, Alkoholen, Ketonen und aromatischen Verbindungen untersucht (Lisa Rösner "Optimierung von Probennahme- und Präkonzentrationsverfahren zum Nachweis volatiler organischer Biomarker in vivo"; Bachelorarbeit Fachhochschule Jena, vorgelegt am 31. August 2011). Um die gewünschten Konzentrationslevel zu erhalten, wurde eine bestimmte Menge von C1-C6-, Aldehyd- und Mix-Standards in Tedlar®-Bags mittels Glasspritze überführt und mit Stickstoff auf 100 ppb beziehungsweise 500 ppb verdünnt. Außerdem wurde eine Atemgasprobe einer Ziege entnommen und die adsorbierte Menge einzelner Substanzen nachgewiesen. Insgesamt zeigen die Ergebnisse, dass bereits geringe Änderungen der Versuchsbedingungen zu deutlich unterschiedlichen Ergebnissen führen.

Mieth et al. (Anal. Chem., 2008, 81, 5851-5857) offenbart die Verwendung von NTDs zur Analyse von VOCs in der Ausatemluft von Schweinen. Dabei wird die Ausatemluft direkt in einer NTD, die die drei Adsorbentien Tenax, Carbopack X und Carboxen enthält, gesammelt. Dabei wird die alveolare Ausatemluft mit einer Geschwindigkeit größer als 60 ml/min während eines Atemzyklus in die NTD aufgezogen. Die Ausatemluft wird mit 2,3-Dimethylbutane, Pentan, Hexan und Heptan versetzt und dann bei verschiedenen Temperaturen (250 Grad Celsius, 285 Grad Celsius und 300 Grad Celsius) desorbiert. Mittels GC/MS wird die desorbierte Menge an 2,3-Dimethylbutane, Pentan, Hexan und Heptan bestimmt.

Die Verwendung von NTDs zum Nachweis von Erkrankungen, wie beispielsweise infektiösen Lungenkrankheiten wurde bisher nicht beschrieben.

Pseudomonas aeruginosa ist ein gram-negatives, stäbchenförmiges Bakterium, welches schwere und zum Teil lebensbedrohliche nosokomiale Infektionen mit einer hohen Mortalität verursacht. Pseudomonas aeruginosa ist ein Keim, der sehr häufig mit Mukoviszidose oder zystische Fibrose (CF) vergesellschaftetet ist. Patienten mit Mukoviszidose oder zystische Fibrose (CF) weisen deshalb oft eine Besiedelung oder Infektion mit Pseudomonas aeruginosa auf, wobei die reine Besiedlung und eine Infektion nur schwer zu unterscheiden sind und eine Infektion deshalb häufig erst im fortgeschrittenen Stadium festgestellt wird.

Mukoviszidose oder zystische Fibrose (CF) ist eine genetisch bedingte, autosomal-rezessive angeborene Stoffwechselerkrankung. Sie ist eine der häufigsten autosomalrezessiven Erbkrankheiten in der Bevölkerung Nordwest-Europas. Durch eine Fehlfunktion von Chloridkanälen verändert sich die Zusammensetzung aller Sekrete exokriner Drüsen mit der Folge eines sehr zähflüssigen Sekrets in den betroffenen Organsystemen. In den Lungen kommt es zur Ausbildung von Bronchiektasen und zur Besiedlung mit Problemkeimen, wie z.B. Pseudomonas aeruginosa. Im Mittel besteht etwa im Alter von 28 Jahren die Indikation zur Lungentransplantation bei ventilatorischem Versagen.

Neben der Freisetzung sogenannter Virulenzfaktoren wie Exotoxine, Proteinasen und Hämolysinen produziert Pseudomonas aeruginosa einen Biofilm, der das zum Teil zerstörte Lungengewebe bei Mukoviszidose Patienten auskleidet. In diesem sind die Erreger in Bezug auf die körpereigene Abwehr als auch durch eine antibiotische Therapie schwer zugänglich. Das ermöglicht Pseudomonas aeruginosa eine fortschreitende Kolonisation der Lunge, was wiederum zu gehäuften respiratorischen Infekten führt. Bis zum heutigen Tag gelingt der Nachweis dieser Bakterien über eine mikrobiologische Kultur oder gentechnische Untersuchung aus dem Sputum oder dem, über eine Bronchoskopie gewonnenen, Bronchialsekret. Beide Verfahren sind zeit- und kostenaufwändig und können die frühe Benennung der zugrundeliegenden Erreger verzögern.

Es besteht deshalb ein Bedarf an neuen, nicht-invasiven und schnellen Verfahren zum Nachweis und zur Verlaufskontrolle bei infektiösen Erkrankungen, insbesondere infektiösen Lungenerkrankungen.

Diese Aufgabe wird erfindungsgemäß durch ein Verfahren gelöst, bei dem die ausgeatmete Luft eines Individuums untersucht wird. Der Vorteil des erfindungsgemäßen Verfahrens besteht darin, dass ganz normal ausgeatmete Luft des zu untersuchenden Individuums aufgefangen wird. Dieses Verfahren ist nichtinvasiv, führt bei dem zu untersuchenden Individuum zu keinerlei Beeinträchtigung und kann beliebig oft wiederholt werden. Das Verfahren ist zudem kostengünstig und liefert im Vergleich zu bisher bekannten Verfahren schnell und zuverlässig reproduzierbare Ergebnisse.

Das erfindungsgemäße Verfahren kann grundsätzlich zum Nachweis und zur Verlaufskontrolle bei allen Erkrankungen eingesetzt werden.

Gegenstand der Erfindung ist ein Verfahren zum Nachweis einer Erkrankung umfassend die Schritte
a) Auffangen der ausgeatmeten Luft eines Individuums;
b) Anreicherung der flüchtigen organischen Substanzen (VOCs) aus der ausgeatmeten Luft in einer Needle Trap (NTD);
c) Nachweis mindestens einer VOC, wobei die VOC für die betreffende Erkrankung spezifisch ist oder der Nachweis von mindestens zwei VOCs, wobei eine Kombination der VOCs für die betreffende Erkrankung charakteristisch ist.

Bevorzugt ist die Anwendung des erfindungsgemäßen Verfahrens zur Diagnose und / oder zur Verlaufskontrolle bei infektiösen Erkrankungen, insbesondere infektiösen Lungenerkrankungen. Eine bevorzugte Ausführungsform der Erfindung betrifft entsprechende Verfahren.

Bevorzugt ist ebenfalls die Anwendung des erfindungsgemäßen Verfahrens zur Diagnose und / oder zur Verlaufskontrolle bei Tumorerkrankungen. Eine bevorzugte Ausführungsform der Erfindung betrifft entsprechende Verfahren.

Gegenstand der Erfindung ist ein Verfahren zum Nachweis einer Erkrankung, insbesondere einer infektiösen Erkrankung oder einer Tumorerkrankung umfassend die Schritte
a) Auffangen der ausgeatmeten Luft eines Individuums;
b) Anreicherung der flüchtigen organischen Substanzen (VOCs) aus der ausgeatmeten Luft in einer Needle Trap Device (NTD);
c) Nachweis mindestens einer VOC, wobei die VOC für die betreffende Erkrankung, insbesondere die infektiöse Erkrankung oder die Tumorerkrankung spezifisch ist.

Die eine einzelne oder die mehreren VOCs können für eine Erkrankung, insbesondere eine infektiöse Erkrankung oder eine Tumorerkrankung spezifisch beziehungsweise charakteristisch sein. In diesem Fall reicht die Bestimmung einer spezifischen VOC zum Nachweis der Erkrankung, beispielsweise der infektiösen Erkrankung oder der Tumorerkrankung aus. Für eine Erkrankung, beispielsweise eine infektiöse Erkrankung oder eine Tumorerkrankung können auch mehrere VOCs spezifisch sein. Zum Nachweis einer Erkrankung, beispielsweise einer infektiösen Erkrankung können gegebenenfalls mehrere, beispielsweise zwei bis fünf oder mehr, vorzugsweise drei oder vier spezifische VOCs bestimmt werden.

Ein VOC, das für eine infektiöse Erkrankung spezifisch ist, wird in der Ausatemluft des Individuums nur dann nachgewiesen, wenn eine Infektion oder Besiedelung mit dem die betreffende infektiöse Erkrankung auslösenden Erreger vorliegt. In diesem Fall besteht eine Korrelation zwischen dem nachgewiesen spezifischen VOC und dem die infektiöse Erkrankung auslösenden Erreger, d.h. wird das oder die spezifische VOC nachgewiesen, dann kann daraus die Diagnose abgeleitet werden, dass eine Besiedelung oder Infektion mit dem die infektiöse Erkrankung auslösenden Erreger vorliegt. Das schließt nicht aus, dass es möglicherweise andere Erreger gibt, die in der Ausatemluft des Individuums ebenfalls dieses spezifische VOC verursachen würden, jedoch rufen diese anderen Erreger nicht die mit der betreffenden infektiösen Erkrankung assoziierten Symptome bei dem Individuum hervor und sind dadurch eindeutig von dem die infektiöse Erkrankung auslösenden Erreger zu unterscheiden.

Eine Erkrankung, beispielsweise eine infektiöse Erkrankung kann auch mit Hilfe von mindestens zwei, vorzugsweise 3 bis 10 oder mehr, beispielsweise vier, fünf, sechs, sieben, acht oder neun unspezifischen VOCs nachgewiesen werden. Die einzelnen VOCs treten dabei bei verschiedenen Erregern auf und rufen gleiche, ähnliche oder teilweise gleiche Symptome einer infektiösen Erkrankung hervor, so dass die einzelnen unspezifischen VOCs nicht für eine bestimmte infektiöse Erkrankung spezifisch sind. Der Nachweis von zwei oder mehr VOCs, d.h. das kombinierte Auftreten von zwei oder mehr unspezifischen VOCs, ist jedoch charakteristisch für einen bestimmten Erreger und damit für eine bestimmte infektiöse Erkrankung. Die Anzahl der benötigten unspezifischen VOCs zum Nachweis eines definierten Erregers können vom Fachmann ermittelt werden.

Gegenstand der Erfindung ist deshalb auch ein Verfahren zum Nachweis einer Erkrankung, insbesondere einer infektiösen Erkrankung oder einer Tumorerkrankung umfassend die Schritte
a) Auffangen der ausgeatmeten Luft eines Individuums;
b) Anreicherung der VOCs aus der ausgeatmeten Luft in einer NTD;
c) Nachweis von mindestens zwei unspezifischen VOCs, wobei der Nachweis der Kombination der unspezifischen VOCs für die betreffende Erkrankung, insbesondere die infektiöse Erkrankung oder die Tumorerkrankung spezifisch ist.

Eine Erkrankung, beispielsweise eine infektiöse Erkrankung kann auch mit Hilfe einer Kombination aus spezifischen und unspezifischen VOCs nachgewiesen werden. Dabei kann ein Nachweis von ein oder mehreren spezifischen und ein oder mehreren unspezifischen VOCs nacheinander oder gleichzeitig erfolgen, um den Erreger der Erkrankung, beispielsweise der infektiösen Erkrankung zu ermitteln.

Gegenstand der Erfindung ist deshalb auch ein Verfahren zum Nachweis einer Erkrankung, insbesondere einer infektiösen Erkrankung oder einer Tumorerkrankung umfassend die Schritte
a) Auffangen der ausgeatmeten Luft eines Individuums;
b) Anreicherung der VOCs aus der ausgeatmeten Luft in einer NTD;
c) Nachweis von mindestens einem spezifischen VOC und mindestens einem unspezifischen VOC und wobei die Kombination aus spezifischem VOC und unspezifischem VOC für eine Erkrankung, insbesondere eine infektiöse Erkrankung oder eine Tumorerkrankung spezifisch ist.

Bei jeder Erkrankung, beispielsweise infektiösen Erkrankung eines Individuums ändert sich die Zusammensetzung der ausgeatmeten VOCs. Durch die Infektion mit Erregern werden Stoffwechselprodukte erzeugt, die ein oder mehrere flüchtige organische Substanzen umfassen, die entweder einzeln oder in Kombination spezifisch für die betreffende infektiöse Erkrankung, die durch den Erreger ausgelöst wird, sind.

Infektiöse Erkrankungen, auch Infektion oder Infektionskrankheiten genannt, werden durch das Eindringen, Verbleiben und die gegebenenfalls anschließende Vermehrung von Erregern verursacht.

Erreger sind Stoffe oder Organismen, die in dem Individuum gesundheitsschädigende Abläufe verursachen. Erreger können pathogene Lebewesen oder pathogene Moleküle sein. Beispiele für Erreger sind Bakterien, Viren, Viroide, Virusoide, Parasiten, Protisten, Prionen, Pilze, Algen, Transposons, Retroposons, Retroviroide, Satelliten-DNA.
Konkrete Beispiele für Erreger sind Pneumonien, VAP (ventilator associated pneumonia), Pseudomonas aeruginosa, Staphylococcus aureus, Streptococcus pneumoniae, Enterococcus faecalis, Klebsiella pneumoniae, Escherichia coli.

In einer Ausführungsform der Erfindung ist die infektiöse Erkrankung eine infektiöse Lungenerkrankung, beispielsweise eine infektiöse Lungenerkrankung mit einem der Erreger ausgewählt aus Pneumonien, VAP (ventilator associated pneumonia), Pseudomonas aeruginosa, Staphylococcus aureus, Streptococcus pneumoniae, Enterococcus faecalis, Klebsiella pneumoniae, Escherichia coli.

In einer anderen Ausführungsform der Erfindung ist die Erkrankung eine Tumorerkrankung, beispielsweise infektiöse Tumorerkrankung, beispielsweise Lungenkrebs, Leberkrebs, Magenkrebs, Brustkrebs, Melanom.

VOCs, die charakteristisch für eine infektiöse Erkrankung sind, umfassen alle flüchtigen organischen Substanzen, die bei der betreffenden infektiösen Erkrankung durch den die infektiöse Erkrankung verursachenden Erreger auftreten, vermehrt auftreten, weniger auftreten oder nicht mehr auftreten. Dies können ein oder mehrere flüchtige organische Substanzen sein. Die VOCs müssen dabei nicht insofern spezifisch sein, dass sie nur bei einem einzigen spezifischen Keim auftreten. Vielmehr sind auch VOCs charakteristisch beziehungsweise spezifisch für eine infektiöse Erkrankung, die bei mehreren unterschiedlichen Keimen gebildet werden. Es können ein oder mehrere VOCs in der Ausatemluft eines Individuums enthalten sein, die für eine infektiöse Erkrankung spezifisch sind. In einer besonderen Ausführungsform der Erfindung ist bereits ein VOC ausreichend spezifisch für die infektiöse Erkrankung, beispielsweise 1-Undecen für eine Infektion oder Besiedelung mit dem Bakterium Pseudomonas aeruginosa. In einer anderen besonderen Ausführungsform der Erfindung kann die infektiöse Erkrankung durch das Auftreten von zwei oder mehr, beispielsweise drei, vier oder fünf oder mehr VOCs nachgewiesen werden. Sofern es sich um unspezifische VOCs handelt, ist die Kombination der unspezifischen VOCs charakteristisch für die betreffende infektiöse Erkrankung.

Erfindungsgemäß bevorzugt ist der Nachweis einer Infektion oder Besiedelung mit Pseudomonas aeruginosa. Eine Infektion beziehungsweise Besiedelung mit Pseudomonas aerguinosa kann durch das Auftreten von einem oder mehreren der VOCs ausgewählt aus 1-Undecen, 2-Butanon, Dimethyldisulfid, Dimethyltrisulfid, 2-Butanon, 2-Aminoacetophenon, 2-Nonanon nachgewiesen werden. Einen besonderen Stellenwert nimmt dabei das 1-Undecene ein, da es bisher bei keinem anderen Erreger nachgewiesen wurde. 1-Undecene ist somit ein spezifisches VOC für eine infektiöse Erkrankung, die durch den Erreger Pseudomonas aerguinosa verursacht ist.

Eine besonders bevorzugte Ausführungsform der Erfindung betrifft ein Verfahren zum Nachweis einer Infektion oder Besiedelung mit Pseudomonas aeruginosa umfassend die Schritte
a) Auffangen der ausgeatmeten Luft eines Individuums;
b) Anreicherung der VOCs aus der ausgeatmeten Luft in einer NTD;
c) Nachweis eines oder mehrerer VOCs ausgewählt aus 1-Undecen, 2-Butanon, Dimethyldisulfid, Dimethyltrisulfid, 2-Butanon.

Desweiteren kann bei Anwesenheit einer oder mehrerer der VOCs ausgewählt aus 3-Methylbutanal, Acetoin, 3-Methyl-1-butanal und Isovaleriansäure auf eine infektiöse Erkrankung mit dem Erreger Staphylococcus aureus geschlossen werden. Eines oder mehrere der VOCs ausgewählt aus Methanol, Pentanol und Ethylacetat weisen auf eine infektiöse Erkrankung mit dem Erreger Escherichia coli hin.

Eine besonders bevorzugte Ausführungsform der Erfindung betrifft ein Verfahren zum Nachweis einer Infektion oder Besiedelung mit Staphylococcus aureus umfassend die Schritte
a) Auffangen der ausgeatmeten Luft eines Individuums;
b) Anreicherung der VOCs aus der ausgeatmeten Luft in einer NTD;
c) Nachweis eines oder mehrerer VOCs ausgewählt aus 3-Methylbutanal, Acetoin, 3-Methyl-1-butanal, Isovaleriansäure.

Eine besonders bevorzugte Ausführungsform der Erfindung betrifft ein Verfahren zum Nachweis einer Infektion oder Besiedelung mit Escherichia coli umfassend die Schritte
a) Auffangen der ausgeatmeten Luft eines Individuums;
b) Anreicherung der VOCs aus der ausgeatmeten Luft in einer NTD;
c) Nachweis eines oder mehrerer VOCs ausgewählt aus Methanol, Pentanol, Ethylacetat.

Lungenkrebs kann durch ein oder mehrere der VOCs ausgewählt aus Isoproyplalkohol, 4-Pentenol, 2,3 Hexandion, 1,1,2-trichloro-1,2,2-trifluoroethan nachgewiesen werden. Brustkrebs kann durch ein oder mehrere der VOCs ausgewählt aus Acetophenon und Heptanal nachgewiesen werden. Magenkrebs kann durch ein oder mehrere der VOCs ausgewählt aus 1-Propanol und Carbondisulfid nachgewiesen werden.

Eine besonders bevorzugte Ausführungsform der Erfindung betrifft ein Verfahren zum Nachweis von Lungenkrebs umfassend die Schritte
a) Auffangen der ausgeatmeten Luft eines Individuums;
b) Anreicherung der VOCs aus der ausgeatmeten Luft in einer NTD;
c) Nachweis eines oder mehrerer VOCs ausgewählt aus Isoproyplalkohol, 4-Pentenol, 2,3 Hexandion, 1,1,2-trichloro-1,2,2-trifluoroethan.

Eine besonders bevorzugte Ausführungsform der Erfindung betrifft ein Verfahren zum Nachweis von Brustkrebs umfassend die Schritte
a) Auffangen der ausgeatmeten Luft eines Individuums;
b) Anreicherung der VOCs aus der ausgeatmeten Luft in einer NTD;
c) Nachweis eines oder mehrerer VOCs ausgewählt aus Acetophenon, Heptanal.

Eine besonders bevorzugte Ausführungsform der Erfindung betrifft ein Verfahren zum Nachweis von Magenkrebs umfassend die Schritte
a) Auffangen der ausgeatmeten Luft eines Individuums;
b) Anreicherung der VOCs aus der ausgeatmeten Luft in einer NTD;
c) Nachweis eines oder mehrerer VOCs ausgewählt aus 1-Propanol und Carbondisulfid.

Tuberkulose kann durch das VOC 1,3 Isobenzofurandion nachgewiesen werden.

Eine besonders bevorzugte Ausführungsform der Erfindung betrifft ein Verfahren zum Nachweis von Tuberkulose umfassend die Schritte
a) Auffangen der ausgeatmeten Luft eines Individuums;
b) Anreicherung der VOCs aus der ausgeatmeten Luft in einer NTD;
c) Nachweis von 1,3 Isobenzofurandion.

Zur Durchführung des Verfahrens wird die ausgeatmete Luft des Individuums aufgefangen. Beispielsweise atmet das Individuum in eine Glasspritze, die ein entsprechendes Mundstück enthält, beispielsweise als Mundstück eine Olive aus Metall. Die Luft wird beispielsweise für einen Zeitraum von 10 Minuten aufgefangen. Die Glasspritze hat vorzugsweise ein Volumen von 50-100 ml.

Dabei wird die exhalierte Luft vorzugsweise im Rahmen einer normalen Ausatmung aufgefangen und der NTD zugeführt. Hierfür wird das Individuum, beispielsweise der Patient gebeten, nach einer normalen Einatmung beispielsweise in eine 100 ml Glasspritze mit einem geschliffenen Kolben über den Luer-Lock Konus so aufzupusten, dass sich der Kolben aufwärts bewegt und sich so die Spritze mit beispielsweise 90-100 ml Ausatemluft füllt. Danach kann die NTD auf die Spritze aufgesetzt und in eine handelsübliche Spritzenpumpe (Perfusor) eingespannt werden. Im Anschluss kann mit einer standardisierten Pumpengeschwindigkeit von beispielsweise 5 bis 20 ml pro Minute, vorzugsweise 5 bis 10 ml pro Minute, besonders bevorzugt 4 bis 6 ml pro Minute oder etwa 5 ml pro Minute, ganz besonders bevorzugt 5 ml pro Minute die asservierte Luft durch die Kohlenstoffnadel geleitet und dort adsorbiert werden. Die NTD wird von der Glasspritze dekonnektiert. Sie kann zur Aufbewahrung oder zum Transport mit einer Schutzkappe versehen werden.

In einer Ausführungsform des Verfahrens werden die VOCs dadurch angereichert, dass die ausgeatmete Luft ein oder mehrmals in die NTD aufgezogen wird, beispielsweise 5 bis 30 mal, vorzugsweise 10 bis 20 mal, besonders bevorzugt 15 mal. Die ausgeatmete Luft wird beispielsweise mit einer Flussgeschwindigkeit von 1 ml/min bis 20 ml/min in die NTD aufgezogen, vorzugsweise mit einer Flussgeschwindigkeit von 3 bis 10 ml/min, besonders bevorzugt mit 5 ml/min.

Die NTD kann eine Länge von 20 bis 200 mm haben, vorzugsweise 30 bis 100 mm, besonders bevorzugt 50 mm bis 70 mm. Die NTD hat vorzugsweise einen Durchmesser von mindestens 15 Gauge, vorzugsweise mindestens 20 Gauge, besonders bevorzugt Gauge 22 (0,72 mm/0,4 mm) oder Gauge 23 (0,64 mm/0,35 mm). Die NTD umfasst eine Nadel, vorzugsweise eine Edelstahlnadel 22G (60 mm x 0,41 mm Innendurchmesser x 0,72 mm Außendurchmesser). Die Nadelspritze weist vorzugsweise ein Seitenloch auf, das gerade, konisch oder angeschliffen sein kann. Die NTD weist vorzugsweise außerdem einen Luer-Lock-Anschluss auf. Der Luer-Lock-Anschluss entspricht den gängigen Normen, beispielsweise DIN-Norm DIN 13090, EU-Norm EN 1707:1996, internationale Norm ISO 594/1-1986. Die genauen Abmessungen und Ausführungen der NTD können vom Fachmann den jeweiligen Bedürfnissen ohne großen Aufwand angepasst werden und hängen beispielsweise vom verwendeten Injektortyp des Analysegerätes und dem Behältnis, in dem die ausgeatmete Luft des Individuums aufgefangen wird, ab.

Mit der NTD ist eine Präkonzentration der VOCs möglich. Dabei reichen geringe Volumina an Ausatemluft aus, beispielsweise 5 bis 100 ml ausgeatmeter Luft. Es lassen sich polare sowie unpolare VOCs anreichern. Die Handhabung ist schnell und einfach. Die NTD kann in der Regel mindestens 50 mal wiederverwendet werden. Alternativ sind aber auch Einweg NTDs möglich, beispielsweise in einem Diagnostikum oder einem Test Kit.

Die NTD enthält ein oder mehrere Adsorbentien, vorzugsweise 2 bis 5 Adsorbentien, besonders bevorzugt 3 Adsorbentien. Die einzelnen Adsorbentien können unabhängig voneinander ausgewählt werden beispielsweise aus Polymerfasern, porösen organischen Polymeren wie Tenax®, Divinylbenzen (DVB), Polydimethylsiloxan (PDMS), Graphitkohlenstoffen wie Carbopack, Kohlenstoff-Molekularsieben wie Carbosieve, Carboxen, Co-polymeren wie organischen Lösungsmitteln, Trimethylaminen, Fettsäuren oder Petroleum.

Die absorbierten VOCs werden vorzugsweise thermisch aus der NTD desorbiert, beispielsweise mittels GC-MS. Die Desorption wird beispielsweise bei einer Temperatur von 150 bis 350 Grad Celsius, vorzugsweise 180 bis 320 Grad Celsius, besonders bevorzugt 200 bis 300 Grad Celsius durchgeführt.

Gegenstand der Erfindung ist auch ein Verfahren zum Nachweis einer Erkrankung, insbesondere einer infektiösen Erkrankungen umfassend die Schritte
a) Auffangen der ausgeatmeten Luft eines Individuums;
b) Anreicherung der VOCs aus der ausgeatmeten Luft in einer NTD;
c) Thermische Desorption der angereicherten VOCs aus der NTD in ein oder mehrere Analysegeräte;
d) Nachweis mindestens einer VOC, wobei die VOC für die betreffende Erkrankung, insbesondere die betreffende infektiöse Erkrankung spezifisch ist.

Gegenstand der Erfindung ist auch ein Verfahren zum Nachweis einer Erkrankung, insbesondere einer infektiösen Erkrankungen umfassend die Schritte
a) Auffangen der ausgeatmeten Luft eines Individuums;
b) Anreicherung der VOCs aus der ausgeatmeten Luft in einer NTD;
c) Thermische Desorption der angereicherten VOCs aus der NTD in ein oder mehrere Analysegeräte;
d) Nachweis von mindestens zwei VOCs, wobei die Kombination der VOCs für die betreffende Erkrankung, insbesondere die betreffende infektiöse Erkrankung spezifisch ist.

In einer besonders bevorzugten Ausführungsform werden die VOCs direkt in ein Analysegerät desorbiert. Das Analysegerät kann ein Gaschromatograph (GC) und/oder ein Massenspektrometer (MS) sein. In einer weiteren bevorzugten Ausführungsform des Verfahrens werden die desorbierten VOCs gaschromatographisch aufgetrennt und dann massenspektroskopisch nachgewiesen. In einer anderen Ausführungsform des Verfahrens werden einzelne VOCs durch Antikörper nachgewiesen. Weitere Nachweismöglichkeiten sind dem Fachmann bekannt.

Das Individuum kann ein beliebiges Säugetier sein, vorzugsweise ein Mensch. Das Individuum kann außer der gegebenenfalls nachzuweisenden Erkrankung, beispielsweise der infektiösen Erkrankung oder der Tumorerkrankung keine weitere Erkrankung aufweisen. Andererseits kann das Individuum ein Patient sein, bei dem neben der nachzuweisenden Erkrankung, beispielsweise der infektiösen Erkrankung oder der Tumorerkrankung bereits eine andere Erkrankung nachgewiesen wurde, die als Primärerkrankung bezeichnet wird. In einer besonderen Ausführungsform der Erfindung stehen die Primärerkrankung und die nachzuweisende Erkrankung, beispielsweise die infektiöse Erkrankung oder die Tumorerkrankung in einem unmittelbaren Zusammenhang, wie beispielsweise die Infektion oder chronische Kolonisation (Besiedelung) von CF-Patienten mit Pseudomonas aeruginosa.

Eine weitere besondere Ausführungsformen der Erfindung betrifft Verfahren, wobei das Individuum ein Patient mit einer primären Erkrankung ist.

Gegenstand der Erfindung ist ein Verfahren zum Nachweis einer Erkrankung, beispielsweise einer infektiösen Erkrankungen oder einer Tumorerkrankung bei einem Patienten mit einer Primärerkrankung umfassend die Schritte
a) Auffangen der ausgeatmeten Luft des Patienten;
b) Anreicherung der VOCs aus der ausgeatmeten Luft in einer NTD;
c) Nachweis mindestens einer VOC, wobei die VOC für die Erkrankung, beispielsweise die infektiöse Erkrankung oder die Tumorerkrankung spezifisch ist.

Gegenstand der Erfindung ist auch ein Verfahren zum Nachweis einer Erkrankung, beispielsweise einer infektiösen Erkrankungen oder einer Tumorerkrankung bei einem Patienten mit einer Primärerkrankung umfassend die Schritte
a) Auffangen der ausgeatmeten Luft des Patienten;
b) Anreicherung der VOCs aus der ausgeatmeten Luft in einer NTD;
c) Nachweis von mindestens zwei VOCs, wobei die Kombination der VOCs für die betreffende Erkrankung, beispielsweise die infektiöse Erkrankung oder die Tumorerkrankung spezifisch ist.

Die VOCs können spezifische oder unspezifische VOCs sein und sind wie vorstehend definiert.

Die Primärerkrankung des Patienten kann jede Art von Erkrankung sein. In einer Ausführungsform der Erfindung ist die Primärerkrankung eine Lungenerkrankung, beispielsweise eine Lungenerkrankung ausgewählt aus Zystischer Fibrose (CF)(auch Mukoviszidose genannt), Bronchiektasenerkrankung, Alpha-1-Antitrypsinmangel Emphysem, primäre ciliäre Dyskenesie, chronischer obstruktiver Lungenerkrankung (COPD). Die primäre Erkrankung des Patienten kann auch eine Tumorerkrankung sein.

Eine bevorzugte Ausführungsform der Erfindung betrifft ein Verfahren zum Nachweis einer Infektion oder Besiedelung mit Pseudomonas aeruginosa bei Patienten mit Zystischer Fibrose umfassend die Schritte
a) Auffangen der ausgeatmeten Luft des Patienten;
b) Anreicherung der VOCs aus der ausgeatmeten Luft in einer NTD;
c) Nachweis mindestens einer VOC ausgewählt aus 1-Undecen, 2-Butanon, Dimethyldisulfid, Dimethyltrisulfid, 2-Butanon.

Gegenstand der Erfindung ist auch die Verwendung des erfindungsgemäßen Verfahrens. Das Verfahren kann beispielsweise zur qualitativen oder quantitativen Bestimmung einer oder mehrerer VOC(s) in der Ausatemluft eines Individuums verwendet werden. Das Verfahren kann zur Diagnose einer Erkrankung, insbesondere einer infektiösen Erkrankung, einer chronischen bakteriellen Kolonisation, zur Prognose, zur Verlaufskontrolle, zur Entscheidung über eine geeignete Therapie, zur Therapiesteuerung, zur Überwachung im Rahmen der Nachsorge, zur Risikostratifizierung verwendet werden. Bevorzugt ist die Verwendung bei einer infektiösen Lungenerkrankung. Weiterhin bevorzugt ist die Verwendung bei Patienten mit einer Primärerkrankung, die zusätzlich eine infektiöse Erkrankung haben.

Die erfindungsgemäßen Verfahren können zum qualitativen oder quantitativen Nachweis von VOCs angewendet werden. Durch den quantitativen Nachweis von VOCs kann beispielsweise zwischen einer Infektion und einer reinen Besiedelung, z.B. einer chronischen Kolonialisierung im Rahmen einer infektiösen Erkrankung unterschieden werden. Weiterhin können die erfindungsgemäßen Verfahren zum Nachweis einer infektiösen Erkrankung zu einem bestimmten Zeitpunkt, beispielsweise vor Beginn einer Therapie im Rahmen einer Querschnittsanalyse oder zur Verlaufskontrolle, beispielsweise zu verschiedenen Zeiten während einer Therapie im Rahmen einer Longitudialanalyse eingesetzt werden.

Gegenstand der Erfindung ist auch ein Verfahren zur Verlaufskontrolle (Longitudialanalyse) einer Erkrankung, insbesondere einer infektiösen Erkrankungen bei einem Individuum oder einem Patienten mit einer Primärerkrankung umfassend die Schritte
a) Auffangen der ausgeatmeten Luft des Individuums oder des Patienten zu einem ersten Zeitpunkt, beispielsweise vor Beginn der Therapie;
b) Anreicherung der VOCs aus der ausgeatmeten Luft in einer NTD;
c) Nachweis mindestens einer VOC, wobei die VOC für die Erkrankung, insbesondere die infektiöse Erkrankung spezifisch ist;
d) Auffangen der ausgeatmeten Luft des Individuums oder des Patienten zu einem zweiten Zeitpunkt, beispielsweise nach Beginn der Therapie;
e) Anreicherung der VOCs aus der ausgeatmeten Luft in einer NTD;
f) Nachweis mindestens einer VOC, wobei die VOC für die Erkrankung, insbesondere die infektiöse Erkrankung spezifisch ist;
g) Vergleich der Ergebnisse aus Schritt c) und f).

Gegenstand der Erfindung ist auch ein analoges Verfahren zur Verlaufskontrolle einer Tumorerkrankung.

Gegenstand der Erfindung ist auch ein Verfahren zur Verlaufskontrolle (Longitudialanalyse) einer Erkrankung, insbesondere einer infektiösen Erkrankungen bei einem Individuum oder einem Patienten mit einer Primärerkrankung umfassend die Schritte
a) Auffangen der ausgeatmeten Luft des Individuums oder des Patienten zu einem ersten Zeitpunkt, beispielsweise vor Beginn der Therapie;
b) Anreicherung der VOCs aus der ausgeatmeten Luft in einer NTD;
c) Nachweis von mindestens zwei VOCs, wobei die Kombination der VOCs für die Erkrankung, insbesondere die infektiöse Erkrankung spezifisch ist;
d) Auffangen der ausgeatmeten Luft des Individuums oder des Patienten zu einem zweiten Zeitpunkt, beispielsweise nach Beginn der Therapie;
e) Anreicherung der VOCs aus der ausgeatmeten Luft in einer NTD;
f) Nachweis von mindestens zwei VOCs, wobei die Kombination der VOCs für die Erkrankung, insbesondere die infektiöse Erkrankung spezifisch ist;
g) Vergleich der Ergebnisse aus Schritt c) und f).

Gegenstand der Erfindung ist auch ein analoges Verfahren zur Verlaufskontrolle einer Tumorerkrankung.

Gegenstand der Erfindung ist auch ein Diagnostikum oder ein Test Kit zum Nachweis von Erkrankungen, beispielsweise Tumorerkrankungen oder infektiösen Erkrankungen, vorzugsweise von infektiösen Lungenerkrankungen. Das Diagnostikum oder der Test Kit umfassen mindestens ein Mittel zum Auffangen der ausgeatmeten Luft, beispielsweise eine Spritze mit Mundstück, mindestens eine NTD und gegebenenfalls weitere Hilfs- und Zusatzstoffe. Das Diagnostikum oder der Test Kit kann weiterhin eine Heizvorrichtung zur thermischen Desorption der VOCs umfassen. Das Diagnostikum oder der Test Kit können auch mindestens ein Mittel zum Nachweis mindestens einer VOC umfassen, die für die betreffende Erkrankung, beispielsweise die infektiöse Erkrankung spezifisch ist. Das Mittel zum Nachweis kann beispielsweise einen Antikörper und gegebenenfalls Mittel zum Nachweis eines Bindungserfolges zwischen VOC und Antikörper umfassen.

Die nachfolgenden Beispiele und Figuren dienen der Erläuterung der Erfindung, ohne die Erfindung jedoch auf die Beispiele einzuschränken.

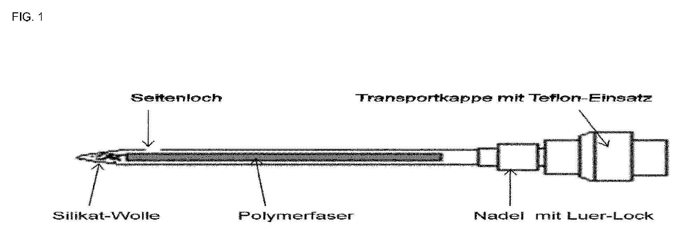

Figur 1 zeigt die schematische Darstellung einer Needle Trap Device (NTD).

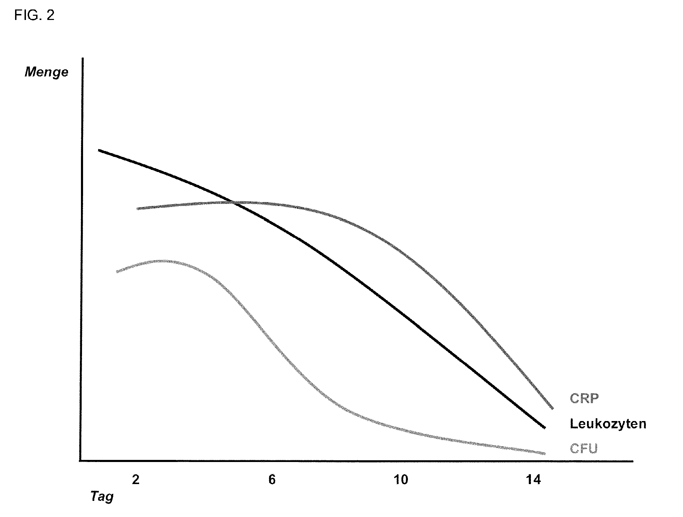

Figur 2 zeigt klinisch relevante Parameter wie das C-reaktive Protein (CRP), die Leukozyten und die Kolonie bildende Einheiten (CFU) über die Zeit von 14 Tagen ohne antibiotische Behandlung des Patienten.

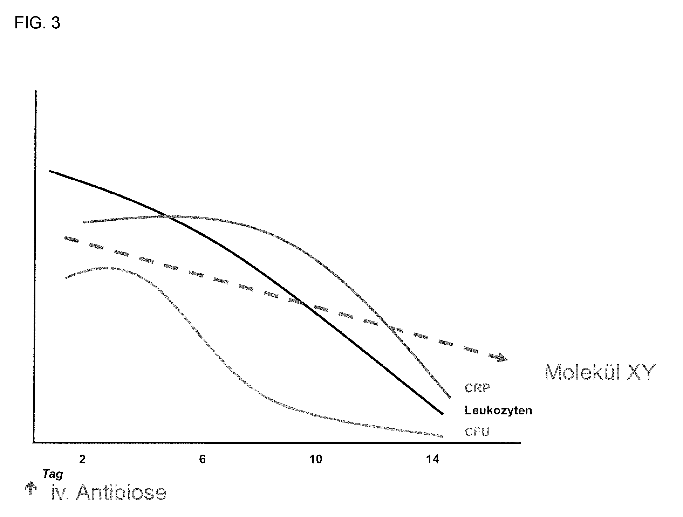

Figur 3 zeigt die Menge der klinischen Parameter CRP, Leukozyten und CFU über die Zeit von 14 Tagen mit antibiotische Behandlung des Patienten. Gezeigt ist auch die durch die antibiotische Therapie verursachte Abnahme des für die betreffende infektiöse Lungenerkrankung spezifischen VOCs (beispielgebend Molekül XY).

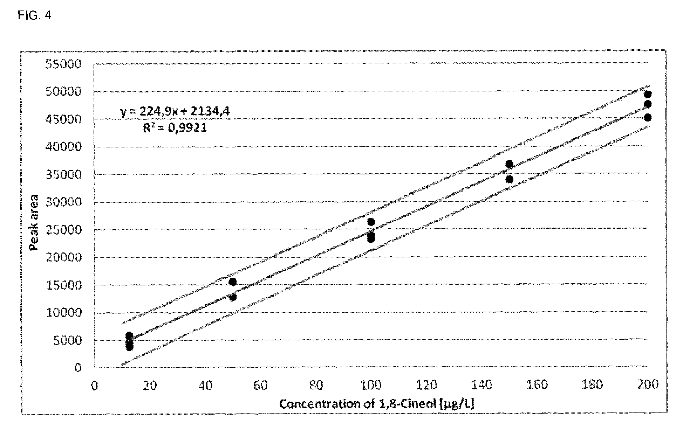

Figur 4 zeigt die Kalibration der NTD mit 1,8-Cineol. Aufgetragen ist die Peakfläche gegen die Konzentration an 1,8-Cineol.

### Beispiele

### Beispiel 1: Zwei-armige Studie

Die Studie umfasst eine qualitative Querschnittsanalyse und quantitative Longitudialanalyse. Die Querschnittsanalyse erlaubt qualitative Aussagen zur Anwesenheit von Pseudomonas aeruginosa Bakterien, durch Nachweis spezifischer volatiler Moleküle im Rahmen eines akuten Infektes. Zum anderen wird durch die Longitudialanalyse eine quantitative Aussage bezüglich dieser initial nachgewiesenen VOCs hinsichtlich des Verlaufes eines respiratorischen Infektes, der durch Pseudomonas aeruginosa verursacht ist, möglich.

### Einschlußkriterien:

Da außerhalb der stationären Routine lediglich die Atemluftasservation erfolgt und dadurch der Patient zu keiner Zeit der Untersuchung beeinträchtigt wird, bestehen keine besonderen Einschlusskriterien. Es besteht lediglich die Bedingung, dass eine schriftliche Einwilligungserklärung für die Atemluftentnahme abgegeben wird.

### Ausschlußkriterien:

Ausschlusskriterien sind schwere Einschränkung der Lungenfunktion, die mit der Unfähigkeit zur Abgabe einer Atemluftspende einhergehen. Weiterhin werden Patienten ausgeschlossen, bei denen eine unklare Erkrankungen und Medikamenteneinnahme gegeben ist oder wenn seitens des Patienten ein Studienteilnahme unerwünscht ist.

### Auswahl der Patienten/Studienprotokoll:

Die Studie beinhaltet mehrere Beobachtungsmessungen pro Patient mit klinischem Hinweis eines respiratorischen Infektes und bereits nachgewiesener Zystischer Fibrose (CF). Desweiteren ist eine anamnestisch bekannte Kolonisierung mit dem Bakterium Pseudomonas aeruginosa (Pa) nachgewiesen. Die Patienten erfahren am Tag 0 keine antibiotische Therapie.

Es können Mehrfachbestimmungen innerhalb eines Tages und/oder innerhalb einer Woche durchgeführt werden. Die Atemluftspende erfolgt morgens vor Gabe eines intravenösen und/oder inhalativen Antibiotikums.

Die Atemluftasservierung erfolgt mittels einer Glasspritze, in die die Patienten Ausatmen. Die Ausatemluft wird in eine NTD überführt, in der die VOCs aus der Ausatemluft des jeweiligen Patienten adsorbiert werden. Die VOCs werden thermisch desorbiert und mittels GC/MS massenspektrometrisch analysiert.

### Beispiel 2: Needle Trap Device (NTD)

Das verwendete NTD umfasst eine Edelstahlnadel 22G (60mm x 0,41mm Innendurchmesser x 0,72mm Aussendurchmesser) mit einer Adsorbtionsspirale aus verschiedenen Kohlenstofffasern und Polymeren wie Tenax®, Carboxen, PDMS , DVB. In der NTD ist eine Präkonzentration der VOCs aus geringen Volumina der Ausatemluft (5-100 ml Ausatemluft) möglich. Mittels der NTD können sowohl polare sowie unpolare VOCs aufgefangen und angereichert werden. Die NTD erlaubt eine schnelle und einfache Handhabung, sie ist wieder verwendbar (50x) und portabel.

### Beispiel 3: Kalibrierung der NTD mit 1,8-Cineol

Es werden 5 wässrige Lösungen (je 30 ml) mit unterschiedlichen Konzentrationen an 1,8-Cineol (12.5 - 200 µg/L 1,8-Cineol) in 400 ml Glasflaschen bei 37°C für 30 min im Wasserbad inkubiert. Je 50 ml Probenvolumen aus dem Gasraum über den 5 wässrigen Lösungen werden mittels einer Glasspritze entnommen und mit einer Geschwindigkeit von 5 ml/min in eine NTD aufgezogen. Das adsorbierte 1,8-Cineol wird in eine GC-MS desorbiert und analysiert.

### Beispiel 4: Atemluftanalyse mit 1,8-Cineol

7 Testpersonen nehmen je eine 1 Kapsel Soledum®. Jede Kapsel Soledum® enthält 200 mg 1,8-Cineol. Eine Atemluftprobe wird sofort nach dem Einnehmen der Soledum® Kapsel (0 Stunden) entnommen (blank). Weitere Atemluftproben werden nach 2 Stunden, 4 Stunden, 6 Stunden und 8 Stunden gesammelt. Um die Atemluftprobe zu gewinnen, atmen die Testpersonen in eine Glasspritze, auf die ein Mundstück, beispielsweise eine Olive aus Metall, aufgesetzt ist, aus. Aus der Glasspritze wird die Atemluft mit einer Geschwindigkeit von 5 ml/min in eine NTD aufgezogen und die in der Atemluft enthaltenen VOCs in der NTD adsorbiert. Die adsorbierten VOCs werden in ein GC-MS Gerät desorbiert und das 1,8-Cineol im Massenspektrum nachgewiesen. Tabelle 1 fasst die allgemeinen Angaben der Testpersonen zusammen, beispielsweise, was die Testpersonen zuvor gegessen haben. Tabelle 2 nennt die Konzentrationen an 1,8-Cineol, die in der Ausatemluft der Testpersonen zu den bestimmten Zeitpunkten nachgewiesen wurden.

**Tabelle 1: Angaben zum allgemeinen Status der Testpersonen**

| **Testperson Nr.** | **Datum** | **Geschlecht** | **Alter** | **Größe [m]** | **Gewicht [kg]** | **BMI** | **Raucher** | **akute Infektion** | **Vorerkrankungen** |
|---|---|---|---|---|---|---|---|---|---|
| 1 | 28.05.2013 | weiblich | 27 | 1.68 | 62 | 22.0 | nein | keine | nein |
| 2 | 03.06.2013 | männlich | 25 | 1.95 | 76 | 20.0 | nein | keine | nein |
| 3 | 04.06.2013 | weiblich | 26 | 1.63 | 62 | 23.3 | ja | keine | nein |
| 4 | 04.06.2013 | männlich | 27 | 1.82 | 62 | 18.7 | ja | keine | nein |
| 5 | 06.06.2013 | männlich | 30 | 1.86 | 62 | 17.9 | ehemaliger | keine | nein |
| 6 | 07.06.2013 | weiblich | 50 | 1.8 | 80 | 24.7 | nein | keine | nein |
| 7 | 07.06.2013 | weiblich | 29 | 1.64 | 70 | 26.0 | gelegentlich | keine | Hypertension |

**Tabelle 2: Messergebnisse der Analyse der Ausatemluft der Testpersonen**

| | **Messzeiten** | | | | | | | | |
|---|---|---|---|---|---|---|---|---|---|
| Test-person Nr. | blank | 1 | 2 | 3 | 4 | Nahrungsaufnahme | Stunde | 1,8-Cineol [µg/L] | |
| 1 | 9:00 | 11:00 | 13:00 | 15:00 | 17:00 | 6:15 Brot mit Nutella, schwarzer Tee; 6:45 Zähne putzen; 11:25 Spinatnudeln mit Ricotta und Käse, rotes Fruchtgelee mit Vanillesauce | 2 | 22.6 | nachweisbar |
| | | | | | | | 4 | 22.1 | nachweisbar |
| | | | | | | | 6 | 21.1 | nachweisbar |
| | | | | | | | 8 | 21.0 | nachweisbar |
| 2 | 10:00 | 12:00 | 14:00 | 16:00 | 18:00 | 7:05 Brot mit Butter und Leberwurst, Apfelsaft; 12:30 Pommes Frites, Currywurst | 2 | 55.3 | 55.3 ± 15.7 µg/L |
| | | | | | | | 4 | 37.7 | nachweisbar |
| | | | | | | | 6 | 34.4 | nachweisbar |
| | | | | | | | 8 | 27.6 | nachweisbar |
| 3 | 10:00 | 12:00 | 14:00 | 16:00 | 18:00 | 8:15 Laugenbrötchen, Kaffee, Espresso 9:55 Kaffee und TicTac; 11:15 Salad, Kartoffelgratin, gebratenes Schwein; 12:00 Kaffee; 13:40 Kaffee | 2 | 15.6 | nachweisbar |
| | | | | | | | 4 | 49.6 | 49.6 ± 15.8 µg/L |
| | | | | | | | 6 | 20.2 | nachweisbar |
| 4 | 9:00 | 11:00 | 13:00 | 15:00 | 17:00 | 8:00 Kaffee, Brot mit Butter und Marmelade; 11:15 Frikandel spezial, Pommes Frites, Salat mit Karotten; 11:45 Kaffee; den ganzen Tag über Wasser | 2 | 51.2 | 51.2 ± 15.7 µg/L |
| | | | | | | | 4 | 31.3 | nachweisbar |
| | | | | | | | 6 | 25.9 | nachweisbar |
| | | | | | | | 8 | 13.2 | nicht nachweisbar |
| 5 | 8:50 | 10:50 | 12:50 | 14:50 | | 7:00 Griesbrei, Brot mit Käse, Gurke, Kaffee; 8:30 Coca Cola; 11:15 Sauerbraten, Knödel, Aprikosen-Pfirsich-Yoghurt | 4 | 50.2 | 50.2 ± 15.8 µg/L |
| 6 | 8:00 | 10:00 | 12:00 | 14:00 | 16:00 | 6:30 Kaffee, Milch, Getreideflocken; 11:30 Wasser, Schweinesteak, Bohnen, Kartoffelbrei, Apfelsaftschorle; till 12:30 Wasser und Kaffee | 4 | 63.4 | 63.4 ± 15.6 µg/L |
| | | | | | | | 6 | 24.3 | nachweisbar |
| | | | | | | | 8 | 19.7 | nachweisbar |
| 7 | 9:00 | 11:00 | 13:00 | 15:00 | 17:00 | 8:30 Wasser, Tabletten gegen | 4 | 45.2 | nachweisbar |
| | | | | | | Hypertonie (Olmetec); 9:30 Brot mit Käse, Yoghurt mit Erdbeeren, Tee; 11:30 Cordon Bleu mit Zucchini Kroketten, Eis; 14:30 Yoghurt mit Erdbeeren; 16:00 Kräutertee | 6 | 22.8 | nachweisbar |

### Beispiel 5: Nachweis von VOCs in der Ausatemluft

### Sputumanalyse

Für die Sputumanalyse wurden zwei Patienten ausgewählt, bei denen Pseudomonas aeruginosa mikrobiologisch nachgewiesen war (1 Patient mit CF, 1 Patient mit Bronchiektasen). Eine Sputumprobe wurde vor Einleitung einer gezielten Antibiose entnommen und auf Cetrimid Agar/Nalidixinsäure (Selektivmedium) ausgestrichen. Das Selektivmedium enthielt kein Tryptophan, so dass die in dem Sputum enthaltenen Pseudomonas aeruginosa Erreger kein 2-Aminoacetophenon (2AA) bilden konnten. Es zeigte sich, dass das Sputum von Patient 1 nicht mukoid war, während das Sputum von Patient 2 mukoid war.

### Headspaceanalyse der mikrobiologischen Kulturen mittels NTD

Von beiden Patienten wurde ebenfalls vor Einleitung einer gezielten Antibiose eine Sputumprobe entnommen. Je 90 ml Volumen aus dem Gasraum (Headspace) der mikrobiologischen Kultur wurden entnommen und mit einer Geschwindigkeit von 5 ml/min durch die NTD einmalig aufgezogen. Die in der NTD adsorbierten VOCs aus der Atemluft der Patienten 1 und 2 wurden anschließend thermisch desorbiert, gaschromatographisch aufgetrennt und massenspektroskopisch analysiert.

Bei Patient 1 wurden in der Ausatemluft folgende VOCs nachgewiesen: Isopren, Dimethylsulfid, Methyl-1-hexanol, 1-Undecen, 1-Ethyl-2-nitrobenzen, 2-Butanon, Styren und Benzaldehyd.

Bei Patient 2 wurden in der Ausatemluft folgende VOCs nachgewiesen: Isopren, Dimethylsulfid, Dimethyldisulfid, Methyl-1-hexanol, 1-Undecen, Dimethyldisulfid, Essigsäure, 2-Butanon, Styren und Benzaldehyd.

1-Ethyl-2-nitrobenzen wurde nur in Patient 1 nachgewiesen; Dimethyldisulfid und Essigsäure nur in Patient 2. Die Atemluftproben beider Patienten enthielten die VOCs Isopren, Dimethylsulfid, Methyl-1-hexanol, 1-Undecen, 2-Butanon, Styren und Benzaldehyd.

Aus der Literatur ist bekannt, dass 1-Undecan, 2-Butanon und Dimethyl(di)sulfid Stoffwechselprodukte von Pseudomonas aeruginosa sind (Labows JN et al. (1980) J. Clin. Microbiol. 12, 521 - 526). Während Dimethyldisulfid und 2-Butanon auch als Stoffwechselprodukte von anderen Erregern auftreten, ist 1-Undecan eine VOC, die nur bei Besiedelung bzw. Infektionen mit Pseudomonas aerguinosa auftritt (Junger et al. (2012) Applied microbiology and biotechnology 93, 2603-2614). In beiden Patienten konnte durch das erfindungsgemäße Verfahren eine Besiedelung bzw. Infektion mit Pseudomonas aeruginosa nachgewiesen werden.

### Beispiel 6: Qualitative Querschnittsanalyse

Zum Zeitpunkt der stationären Erstvorstellung (Tag 0) eines CF-Patienten (Patient mit Mukoviszidose bzw. Cystrischer Fibrose) mit einem akuten respiratorischen Infekt und anamnestisch bekannter Kolonisation mit Pseudomonas aeruginosa sowie vor Einleitung einer antibiotischen Therapie werden Parameter ermittelt, die routinemäßig im Rahmen der stationären Aufnahme erhoben werden. Außerdem wird eine Probe der Ausatemluft mittels NTD entnommen.

Die am Tag 0 aufgenommenen bzw. entnommenen Proben und Parameter sind: anlegen einer Sputumkultur (qualitativ und quantitativ), Blutentnahme inklusive der Bestimmung der Leukozytenzahl, CRP, Procalcitonin (PCT), eine körperliche Untersuchung, Bestimmung der Vitalparameter, Bestimmung der Lungenfunktion (FEV₁), eine Röntgen-Thorax Aufnahme und die Gewinnung von VOCs aus der Ausatemluft der Patienten mittels Needle Trap Device. Diese Parameter dienen dazu, den akuten respiratorischen Infekt in seinem klinischen Ausmaß zu verifizieren sowie einen gesicherten mikrobiologischen Nachweis von Pseudomonas aeruginosa als auch eine Aussage über die Keimzahl in Form von Kolonie bildenden Einheiten (CFU) zu liefern. Die in der NTD adsorbierten VOCs des jeweiligen Patienten werden thermisch desorbiert, gaschromatographisch aufgetrennt und massenspektrometrisch analysiert.

Der Nachweis eines respiratorischen Effekts verursacht durch Pseudomonas aeruginosa kann auch ausschließlich über den Nachweis einer eines für Pseudomonas aeruginosa spezifischen VOCs in der Ausatemluft des Patienten erfolgen, beispielsweise den Nachweis von 1-Undecan.

### Beispiel 7: Quantitative und qualitative Longitudinalanalyse

Die quantitative Verlaufskontrolle einer durch Pseudomonas aeruginosa verursachten Infektion bei CF-Patienten wird untersucht. Um den möglichen Einsatz dieser Nachweismethode zur Erfolgskontrolle einer antibiotischen Therapie zu evaluieren, werden massenspektrometrische Analysen der Ausatemluft im Zeitverlauf einer antibiotischen Therapie durchgeführt. Hierzu werden Messungen vor Beginn der Therapie mit Messungen während einer Woche Therapie (Probennahme der Ausatemluft des Patienten am Tag 2, 4, 6, 8, 10) und bei Abschluss der antibiotischen Therapie (in der Regel Tag 14) durchgeführt. Um diese Messungen mit dem klinischen Verlauf korrelieren zu können, werden die routinemäßigen Laborkontrollen (inklusive Leukozytenzahl, CRP, PCT) am Tag 2, 4, 6, 8 und 10 durchgeführt. An diesen Tagen findet außerdem routinemäßig eine körperliche Untersuchung und die Bestimmung der Vitalparameter statt. Am Tag 10, respektive Tag 14, und somit zum Zeitpunkt der Beendigung einer antibiotischen Therapie erfolgt eine Schlussevaluierung der Therapie und des klinischen Zustandes der Patienten. Dazu werden die Parameter und Prozeduren wie am Tag 0 erneut routinemäßig erhoben: also Sputumkultur (qualitativ, quantitativ), Blutentnahme (inkl. Leukozytenzahl, CRP, PCT), körperliche Untersuchung, Vitalparameter, Atemluftsample mittels Needle Trap Device zur chemischen GC/MS-Analyse, Lungenfunktion (FEV₁) und eine Röntgen-Thorax Kontrolle.

## Patentansprüche

1. Verfahren zum Nachweis einer Erkrankung umfassend die Schritte
a)Auffangen der ausgeatmeten Luft eines Individuums;
b)Anreicherung der flüchtigen organischen Substanzen (VOCs) aus der ausgeatmeten Luft in einer Needle Trap Device (NTD);
c)Nachweis mindestens einer VOC, wobei die VOC für die betreffende Erkrankung spezifisch ist oder Nachweis von mindestens zwei VOCs, wobei die Kombination der VOCs für die betreffende Erkrankung spezifisch ist.

2. Verfahren zur Verlaufskontrolle einer Erkrankungen umfassend die Schritte
a) Auffangen der ausgeatmeten Luft eines Individuums zu einem ersten Zeitpunkt;
b) Anreicherung der VOCs aus der ausgeatmeten Luft in einer NTD;
c) Nachweis mindestens einer VOC, wobei die VOC für die betreffende Erkrankung spezifisch ist oder Nachweis von mindestens zwei VOCs, wobei die Kombination der VOCs für die betreffende Erkrankung spezifisch ist;
d) Auffangen der ausgeatmeten Luft des Individuums zu einem zweiten Zeitpunkt;
e) Anreicherung der VOCs aus der ausgeatmeten Luft des Individuums in einer NTD;
f) Nachweis mindestens einer VOC, wobei die VOC für die betreffende Erkrankung spezifisch ist oder Nachweis von mindestens zwei VOCs, wobei die Kombination der betreffenden VOCs für die betreffende Erkrankung spezifisch ist;
g) Vergleich der Ergebnisse der Nachweise aus c) und f).

3. Verfahren nach einem der vorgehenden Ansprüche, wobei die Erkrankung eine infektiöse Erkrankung oder eine Tumorerkrankung ist.

4. Verfahren nach einem der vorgehenden Ansprüche, wobei das Individuum ein Mensch ohne Primärerkrankung ist.

5. Verfahren nach einem der Ansprüche 1 bis 3, wobei das Individuum ein Patienten mit einer Primärerkrankung ist.

6. Verfahren nach Anspruch 5, wobei die Primärerkrankung eine Lungenerkrankung ist, beispielsweise Mukoviszidose.

7. Verfahren nach einem der vorgehenden Ansprüche, wobei für die infektiöse Erkrankung ein Erreger ursächlich ist.

8. Verfahren nach Anspruch 7, wobei der Erreger ausgewählt ist aus Bakterien, Viren, Viroiden, Virusoiden, Parasiten, Protisten, Prionen, Pilzen, Algen, Transposons, Retroposons, Retroviroiden, Satelliten-DNA, Pneumonien, VAP (ventilator associated pneumonia), Pseudomonas aeruginosa, Staphylococcus aureus, Streptococcus pneumoniae, Enterococcus faecalis, Klebsiella pneumoniae, Escherichia coli.

9. Verfahren nach einem der vorgehenden Ansprüche, wobei die VOCs dadurch angereichert werden, dass die ausgeatmete Luft ein oder mehrmals mit einer Flussgeschwindigkeit von 1 ml/min bis 20 ml/min in die NTD aufgezogen wird.

10. Verfahren nach einem der vorgehenden Ansprüche, wobei ein, zwei, drei, vier oder fünf verschiedene VOCs nachgewiesen werden.

11. Verfahren nach einem der vorgehenden Ansprüche, wobei das Individuum ein Patient mit der Primärerkrankung Mukosviszidose, der Erreger Pseudomonas aeruginosa und das VOC 1-Undecen ist.

12. Verwendung eines Verfahrens nach einem der vorgehenden Ansprüche zur Diagnose, Prognose, Verlaufskontrolle, Entscheidung über eine geeignete Therapie, Therapiesteuerung, Nachsorge, Risikostratifizierung bei einer Erkrankung, vorzugsweise bei einer infektiösen Erkrankung oder einer Tumorerkrankung.

13. Diagnostikum zum Nachweis einer Erkrankung, beispielsweise einer infektiösen Erkrankung oder einer Tumorerkrankung, vorzugsweise einer infektiösen Lungenerkrankungen umfassend ein Mittel zum Auffangen der ausgeatmeten Luft, eine NTD zur Anreicherung von VOCs aus der ausgeatmeten Luft und gegebenenfalls weitere Hilfs- und Zusatzstoffe.

14. Diagnostikum nach Anspruch 13 umfassend mindestens ein Mittel zum Nachweis mindestens einer VOC, die für die betreffende Erkrankung, beispielsweise die infektiöse Erkrankung oder die Tumorerkrankung, vorzugsweise die infektiöse Lungenerkrankung spezifisch ist oder umfassend mindestens zwei Mittel zum Nachweis von mindestens zwei VOCs, deren Kombination für die betreffende Erkrankung, beispielsweise die infektiöse Erkrankung oder die Tumorerkrankung, vorzugsweise die infektiöse Lungenerkrankungen spezifisch ist.

15. Test Kit umfassend ein Diagnostikum nach einem der Ansprüche 13 oder 14.
